# EUROPEAN PATENT SPECIFICATION

(11) **EP 0 789 679 B1**
(45) Date of publication and mention of the grant of the patent: **09.12.1998**
(21) Application number: 95935415.0
(22) Date of filing: 28.09.1995
(51) Int. Cl.: C07C 45/58, C07C 45/80, C07C 47/19, C07C 31/20, C07C 29/141

(54) **PROCESS FOR PREPARING 1,3-ALKANEDIOLS AND 3-HYDROXYALDEHYDES**
VERFAHREN ZUR HERSTELLUNG VON 1,3-ALKANDIOLEN UND 3-HYDROXYALDEHYDEN
PROCEDE DE PREPARATION DE 1,3-ALCANEDIOLS ET DE 3-HYDROXYALDEHYDES

(30) Priority: 30.09.1994 US 316670; 30.09.1994 US 316668
(43) Date of publication of application: 20.08.1997
(73) Proprietor: SHELL INTERNATIONALE RESEARCH MAATSCHAPPIJ B.V., 2596 HR Den Haag (NL)
(72) Inventor: EUBANKS, David, Cleve, Houston, TX 77083 (US); FORSCHNER, Thomas, Clayton, Richmond, TX 77469 (US); POWELL, Joseph, Broun, Houston, TX 77070 (US); SEMPLE, Thomas, Carl, Friendswood, TX 77546 (US); SLAUGH, Lynn, Henry, Houston, TX 77070 (US); THOMASON, Terry, Blane, Houston, TX 77077 (US); WEIDER, Paul, Richard, Houston, TX 77083 (US); MULLIN, Stephen, Blake, Katy, TX 77449 (US)
(86) International application number: EP9503869
(87) International publication number: WO9610551

(56) References cited:
- WO-A-94/18149
- US-A- 4 678 857
- US-A- 5 030 766
- DATABASE WPI Section Ch, Week 8443 Derwent Publications Ltd., London, GB; Class E19, AN 84-267384 & JP,A,59 164 739 ( DAICEL CHEM IND KK) , 17 September 1984

## Description

This invention relates to a process for preparing 1,3-alkanediols and 3-hydroxyaldehydes by hydroformylating an oxirane (1,2-epoxide). In particular, the invention relates to a process for preparing 1,3-propanediol by hydroformylating ethylene oxide in the presence of a Group VIII-based hydroformylation catalyst and hydrogenating the hydroformylation product.

The preparation of 1,3-alkanediols like 1,3-propanediol (PDO) is disclosed in US-A-3 687 981. The process comprises the hydroformylation of an oxirane such as ethylene oxide in the presence of a metallic carbonyl catalyst containing a metal from Group VIII, followed by hydrogenation of the hydroformylation product. The hydroformylation product of that process is the cyclic hemiacetal dimer of 3-hydroxypropanal (HPA), i.e., 2-(2-hydroxyethyl)-4-hydroxy-1,3-dioxane. PDO is of particular interest as intermediate in the production of polyesters for fibres and films.

Despite the publication of this patent in 1972, fibre-grade polyesters based on PDO are as yet not commercially available. Separation of the catalyst from the cyclic hemiacetal produced in US-A-3 687 981, by phase separation, is complicated and inadequate. As a result, the cost for preparing polymer-grade PDO is too high.

In US-A-3 456 017 and US-A-3 463 819 1,3 alkanediols are prepared directly, with only minor amounts of the intermediate hydroformylation product, in the presence of certain phosphine-modified cobalt carbonyl catalysts. Commercialisation of the process of these US patents is ruled out, due to the excessive amounts of catalyst employed therein. Moreover, no clear teaching is provided for optimal recycle of the hydroformylation catalyst. Also in WO 94/18149 phosphine-modified cobalt carbonyl catalysts are used. They are used in a much smaller amount than in the US patents, producing primarily the 3-hydroxyaldehyde. Again no clear teaching is provided for optimal recycle of the hydroformylation catalyst.

Document US-A-4 678 857 discloses the catalytic hydroformylation of allyl alcohol to 4-hydroxy butanal. The product is separated, with high efficiency, from the other reaction components by water extraction.

It would be desirable to prepare 3-hydroxyaldehydes and 1,3-alkanediols selectively and cheaply. It is therefore an object of the invention to provide an economical process for the preparation of 3-hydroxyaldehydes and 1,3-alkanediols in the presence of a hydroformylation catalyst which process allows for the convenient recycle of the catalyst.

Accordingly, the invention provides a process for preparing 1,3-alkanediols and 3-hydroxyaldehydes by hydroformylating an oxirane with carbon monoxide and hydrogen in the presence of one or more Group VIII metal-based hydroformylation catalysts, and in the presence of an organic solvent, wherein the hydroformylation product is separated by extraction with an aqueous liquid under a carbon monoxide atmosphere.

The oxirane comprises an organic compound, two carbon atoms of which are connected by an oxy linkage as well as by a carbon-carbon single bond. In general terms, the oxiranes comprise hydrocarbyl-epoxides, having at least 2, preferably up to 30, more preferably up to 20, most preferably up to 10 carbon atoms. The hydrocarbyl group may be aryl, alkyl, alkenyl, aralkyl, cycloalkyl, or even alkylene; straight chain or branched chain. Suitable examples of oxiranes include 1,2-epoxyl(cyclo)alkanes, such as ethylene oxide, propylene oxide, 1,2-epoxyoctane, 1,2-epoxycyclohexane, 1,2-epoxy-2,4,4-trimethylhexane, and 1,2-epoxyalkenes such as 1,2-epoxy-4-pentene. Ethylene oxide and propylene oxide are preferred. In view of the demand for PDO, ethylene oxide (EO) is the oxirane most preferably used in the process of the invention.

The hydroformylation reaction is carried out in a liquid solvent inert to the reactants and products, i.e., that is not consumed during the reaction. Upon completion of the reaction, the liquid solvent facilitates the separation of the hydroformylation product. In general, ideal solvents for the hydroformylation process will (a) exhibit low to moderate polarity such that the 3-hydroxyaldehyde will be dissolved to a concentration of at least about 5 wt% under hydroformylation conditions, while significant solvent will remain as a separate phase upon extraction with the aqueous liquid, (b) dissolve carbon monoxide, and (c) be essentially non-water-miscible. By "essentially non-water-miscible" is meant that the solvent has a solubility in water at 25 °C of less than 25 wt% so as to form a separate hydrocarbon-rich phase upon extraction of the 3-hydroxyaldehyde from the hydroformylation reaction mixture. Preferably, this solubility is less than 10 wt%, most preferably less than 5 wt%. The solubility of carbon monoxide in the selected solvent will generally be greater than 0.15 v/v (1 atm, 25 °C), preferably greater than 0.25 v/v, expressed in terms of Ostwald coefficients.

The preferred class of solvents are alcohols and ethers which can be described according to the formula (1)

R¹-O-R² (1)

in which R¹ is selected from hydrogen, a linear, branched, cyclic or aromatic C₁₋₂₀ hydrocarbyl or mono-or polyalkylene oxide and R² is selected from a linear, branched, cyclic or aromatic C₁₋₂₀ hydrocarbyl, alkoxy or mono- or polyalkylene oxide, or R¹, R² and O together form a cyclic ether. The most preferred hydroformylation solvents can be described by the formula (2) 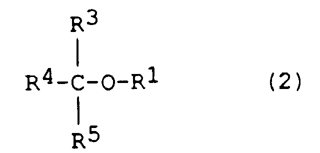 in which R¹ is selected from hydrogen or a C₁₋₈ hydrocarbyl and R³, R⁴ and R⁵ are independently selected from a C₁₋₈ hydrocarbyl, alkoxy or mono- or polyalkylene oxide. Such ethers include, for example, tetrahydrofuran, methyl-t-butyl ether, ethyl-t-butyl ether, ethoxyethyl ether, phenylisobutyl ether, diethyl ether, diphenyl ether, and diisopropyl ether. Blends of solvents such as t-butylalcohol/hexane, tetrahydrofuran/toluene and tetrahydrofuran/heptane can also be used to achieve the desired solvent properties. The currently preferred solvent, because of the high yields of HPA which can be achieved under moderate reaction conditions, is methyl-t-butyl ether.

The hydroformylation reaction is conducted in the presence of any metallic carbonyl hydroformylation catalyst. These catalysts are transition metals, particularly those metals of Group VIII of the Periodic Table, e.g., cobalt, iron, nickel, osmium and complexes described, for example, in US-A-3 161 672. Best results, however, are obtained when a cobalt-based catalyst is used, unmodified cobalt carbonyl compounds being preferred.

The cobalt-based catalyst can be supplied to the hydroformylation reactor as a cobalt carbonyl such as dicobaltoctacarbonyl or cobalt hydridocarbonyl. It may also be supplied in essentially any other form including metal, supported metal, Raney-cobalt, hydroxide, oxide, carbonate, sulfate, acetylacetonate, salt of a fatty acid, or aqueous cobalt salt solution. If not supplied as cobalt carbonyl, operating conditions should be adjusted such that cobalt carbonyls are formed, for instance via reaction with H₂ and CO as described in J. Falbe, "Carbon Monoxide in Organic Synthesis," Springer-Verlag, NY (1970). Typically, these conditions will include a temperature of at least 50 °C and a carbon monoxide partial pressure of at least 0.8 MPa (100 psig). For more rapid reaction, temperatures of 120 to 200 °C should be employed, at CO pressures of at least 3.5 MPa (500 psig). Addition of high surface area activated carbons or zeolites, especially those containing or supporting platinum or palladium metal, is known to accelerate the formation of cobalt carbonyls.

The catalyst is preferably maintained under a stabilising atmosphere of carbon monoxide, which also provides protection against exposure to oxygen. The most economical and preferred catalyst activation and reactivation (of recycled catalyst) method involves converting the cobalt salt (or derivative) under H₂/CO in the presence of the catalyst promoter employed for hydroformylation. The conversion of Co²⁺ to the desired cobalt carbonyl is carried out at a temperature within the range of 75 to 200 °C, preferably 100 to 140 °C and a pressure within the range of 7.0 to 34.6 MPa (1000 to 5000 psig) for a time preferably less than about 3 hours. The preforming step can be carried out in a pressurised preforming reactor or in-situ in the hydroformylation reactor.

The amount of Group VIII metal present in the reaction mixture will vary depending upon the other reaction conditions, but will generally fall within the range of 0.01 wt% to 1 wt%, preferably 0.05 to 0.3 wt%, based on the weight of the reacticn mixture.

The hydroformylation reaction mixture will preferably include a catalyst promoter to accelerate the reaction rate. The promoter will generally be present in an amount within the range of 0.01 to 0.6 moles per mole of Group VIII metal.

Suitable promoters include sources of mono- and multivalent metal cations of weak bases such as alkali, alkaline earth and rare earth metal salts of carboxylic acids. Suitable metal salts include sodium, potassium and caesium acetates, propionates and octoates; calcium carbonate and lanthanum acetate. Also suitable are lipophilic promoters such as lipophilic phosphonium salts, lipophilic amines and -at the aforementioned concentrations- lipophilic bidentate phosphines, which accelerate the rate of hydroformylation without imparting hydrophilicity (water solubility) to the active catalyst. As used herein, "lipophilic" means that the promoter tends to remain in the organic phase after extraction of HPA with water. Suitable lipophilic promoters include e.g., tetra(n-butyl)phosphonium acetate, nonylpyridine, and bidentate diphosphines represented by formula (3) 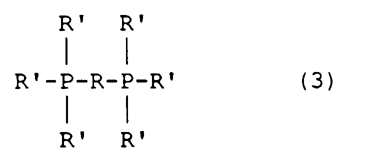 in which R is a C₁₋₃ divalent hydrocarbyl moiety and each R' is independently selected from a linear, branched, cyclic or aromatic C₁₋₂₅ hydrocarbyl, alkoxy or mono-or polyalkenyl oxide, or in which two or more of the R' groups together form a ring structure. Such bidentate phosphines include bis(diphenylphosphino)methane, 1,2-bis(diphenylphosphino)ethane, 1,2-bis(9-phosphabicyclo-[3,3,1 or 4,2,1]nonyl)ethane, and 1,2-bis(dicyclohexylphosphino)ethane.

It is generally preferred to regulate the concentration of water in the hydroformylation reaction mixture, as excessive amounts of water reduce the selectivity towards the 1,3-alkanediols and 3-hydroxyaldehydes below acceptable levels and may induce formation of a second liquid phase. At low concentrations, water can assist in promoting the formation of the desired cobalt carbonyl catalyst species. Acceptable water levels will depend upon the solvent used, with more polar solvents generally more tolerant of higher water concentrations. For example, optimum water levels for hydroformylation in methyl-t-butyl ether solvent are believed to be within the range of 1 to 2.5 wt%.

The hydrogen and carbon monoxide will generally be introduced into the reaction vessel in a molar ratio within the range of 1:2 to 8:1, preferably 1:1.5 to 5:1.

The reaction is carried out under conditions effective to produce a hydroformylation reaction mixture comprising a major portion of the 3-hydroxyaldehyde and a minor portion of by-product, if any. Moreover, the level of 3-hydroxyaldehyde in the reaction mixture is preferably maintained at less than 15 wt%, preferably 5 to 10 wt%. (To provide for solvents having different densities, the concentration of 3-hydroxyaldehyde in the reaction mixture can be expressed in molarity, i.e., less than 1.5M, preferably within the range of 0.5 to 1M.).

Generally, the hydroformylation reaction is carried out at elevated temperature less than 100 °C, preferably 60 to 90 °C, most preferably 75 to 85 °C, and at a pressure within the range of 3.5 to 34.6 MPa (500 to 5000 psig), preferably (for process economics) 7.0 to 24.2 MPa (1000 to 3500 psig), with higher pressures generally imparting greater selectivity. The concentration of 3-hydroxyaldehyde in the intermediate product mixture can be controlled by regulation of process conditions such as oxirane concentration, catalyst concentration, reaction temperature and residence time. In general, relatively low reaction temperatures (below 100 °C) and relatively short residence times within the range of 20 minutes to 1 hour are preferred.

In the practice of the invention method, it is possible to achieve 3-hydroxyaldehyde yields (based on the oxirane conversion) of greater than 80%. For instance, with the hydroformylation of EO in the presence of a cobalt carbonyl, formation of more than 7 wt% HPA in the dilute hydroformylation product mixture, at rates greater than 30 h⁻¹ are achievable. (Catalytic rates are referred to herein in terms of "turnover frequency" or "TOF" and are expressed in units of moles per mole of cobalt per hour, or h⁻¹.) Reported rates are based on the observation that, before a majority of the oxirane, here EO, is converted, the reaction is essentially zero-order in EO concentration and proportional to cobalt concentration.

As mentioned above, separation of the hydroformylation product mixture is carried out with an aqueous liquid.

Preferably the aqueous liquid is water. The amount of water added to the hydroformylation reaction product mixture will generally be such as to provide a weight ratio of water:mixture within the range of 1:1 to 1:20, preferably 1:5 to 1:15. The addition of water at this stage of the reaction may have the additional advantage of suppressing formation of undesirable heavy ends.

Extraction with a relatively small amount of water provides an aqueous phase which is greater than 20 wt% 3-hydroxyaldehyde, preferably greater than 35 wt% 3-hydroxyaldehyde, permitting economical hydrogenation of the 3-hydroxyaldehyde to the 1,3-alkanediol. The water extraction is preferably carried out at a temperature within the range of 25 to 55 °C, with higher temperatures avoided to minimise condensation products (heavy ends) and catalyst disproportionation to inactive, watersoluble Group VIII metal (e.g., cobalt) compounds.

According to the invention, to maximise catalyst recovery, the water extraction is carried out under carbon monoxide. The carbon monoxide can be introduced into the extraction vessel separately or the extraction can be carried out under residual carbon monoxide from the hydroformylation reaction. Suitably, it is carried out under a carbon monoxide partial pressure that is less than that maintained for hydroformylation. Extraction of the 3-hydroxyaldehyde under carbon monoxide enables 80% or more of the hydroformylation catalyst to be extracted into the organic phase. The hydroformylation catalyst may subsequently be recycled to the hydroformylation reaction via a solvent recycle. The (partial) pressure of carbon monoxide is preferably maintained within the range of 0.2 to 13.9 MPa (20 to 2000 psig), most preferably 0.5 to 1.5 MPa (50 to 200 psig). The carbon monoxide may be combined with hydrogen or another inert gas, such as nitrogen, methane or argon.

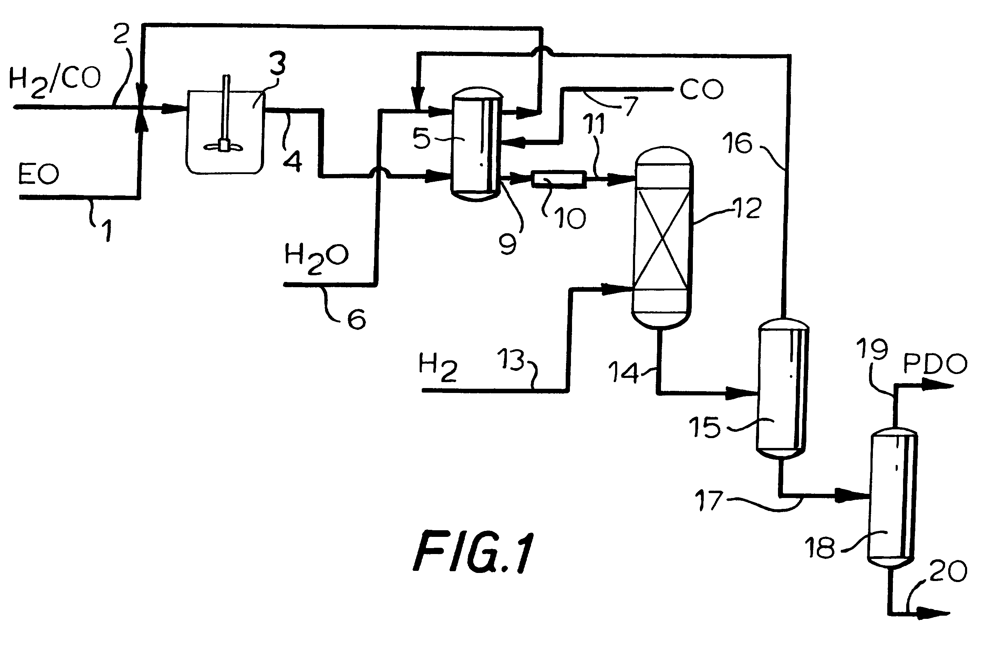

The invention process can be conveniently described by reference to Figure 1. By way of example, the hydroformylation of EO as oxirane will be described. Separate or combined streams of EO *(1),* carbon monoxide and hydrogen *(2)* are charged to hydroformylation vessel *(3)*, which can be a pressure reaction vessel such as a bubble column or agitated tank, operated batch-wise or in a continuous manner. The feed streams are contacted in the presence of an unmodified cobalt-based catalyst, i.e., a cobalt carbonyl compound which has not been prereacted with a phosphine ligand.

Following the hydroformylation reaction, the hydroformylation reaction product mixture *(4)* containing HPA, the reaction solvent, PDO, the cobalt catalyst and a minor amount of reaction by-products, is passed to extraction vessel *(5)*, to which an aqueous liquid, generally water and optionally a miscible solvent, are added via *(6)* for extraction and concentration of the HPA for the subsequent hydrogenation step. Liquid extraction can be effected by any suitable means, such as mixer-setters, packed or trayed extraction columns or rotating disk contactors. Extraction can if desired be carried out in multiple stages. The water-containing hydroformylation reaction product mixture can be passed to a settling tank (not shown) for resolution into aqueous and organic phases.

The organic phase containing the reaction solvent and the major portion of the cobalt catalyst can be recycled from the extraction vessel to the hydroformylation reaction via *(7)*. Aqueous extract *(8)* is optionally passed through one or more acid ion exchange resin beds *(9)* for removal of any cobalt catalyst present, and the decobalted aqueous product mixture *(10)* is passed to hydrogenation vessel *(11)* and reacted with hydrogen *(12)* in the presence of a hydrogenation catalyst to produce a hydrogenation product mixture *(13)* containing PDO. The hydrogenation step may also revert some heavy ends to PDO. The solvent and extractant water *(15)* can be recovered by distillation in column *(14)* and recycled to the water extraction process via a further distillation (not shown) for separation and purge of light ends. PDO-containing stream *(16)* can be passed to one or more distillation columns *(17)* for recovery of PDO *(18)* from heavy ends *(19)*.

The invention process permits the selective and economical synthesis of PDO at moderate temperatures and pressures without the use of a phosphine ligand for the hydroformylation catalyst. The process involves preparation of a reaction product mixture dilute in HPA, then concentration of this HPA by water extraction for subsequent hydrogenation of HPA to PDO.

### Example 1 (COMPARATIVE)

This example illustrates cobalt catalyst recovery under a nitrogen atmosphere during water extraction of HPA from a hydroformylation reaction product mixture. 13g of ethylene oxide were hydroformylated at 80 °C under 10.4 MPa (1500 psig) of 2.3:1 H₂/CO, in an unpromoted reaction mixture containing 0.87g dicobaltoctacarbonyl, 1.5g toluene (internal marker) and 147.5g methyl-t-butyl ether (MTBE). After 5.75 hours of reaction, 4.7 wt% HPA was observed in solution. The reaction mixture was cooled to 25 °C and extracted under nitrogen with 30g of deionised water. 70.56g of the upper organic layer (OL) containing 901 ppm cobalt and 31.85g of the lower aqueous layer (AL) containing 1828 ppm cobalt were isolated. Thus, 52% of the cobalt remained with the organic layer following water extraction.

After removal of the organic layer from the reaction, 0.43g of a 36% acetic acid/water solution were added to the lower aqueous layer along with 150 ml ambient pressure air and a 0.8 MPa (100 psig) nitrogen blanket. The mixture was stirred for 30 min. at room temperature, to attempt to oxidise Co⁻¹, present as tetracarbonyl anion, to dicobaltoctacarbonyl. The resulting oxidation reaction mixture was treated with 26.9g of fresh MTBE to extract any oil-soluble dicobaltoctacarbonyl. No cobalt was extracted into the ether phase.

### Example 2

This example illustrates cobalt catalyst recovery under a carbon monoxide atmosphere during water extraction of HPA from a promoted hydroformylation reaction product mixture. 10g of ethylene oxide were hydroformylated in a reaction mixture containing 0.87g dicobaltoctacarbonyl, 0.14g sodium acetate trihydrate promoter, 1.5g toluene and 1.5g undecanol (internal markers), and 146g MTBE at 80 °C and 10.4 MPa (1500 psig) of 2.3:1 H₂/CO. Analysis after 2 hours 12 minutes of reaction indicated 7.9 wt% HPA in the reaction product mixture.

After removal of a large fraction of this reaction mixture for alternate studies, 51g of the remaining reaction mixture were placed in the reactor and cooled to 27 °C. The reactor was vented and recharged with 1.5 MPa (200 psig) of CO. 16g of water were added to extract HPA. After transferring a majority of the mixture to a phase separator under 1.5 MPa (200 psig) carbon monoxide, 41.2g of upper organic layer were isolated and found to contain 1893 ppm cobalt. 12.9g of the aqueous layer contained 1723 ppm cobalt. 78% of the cobalt present was thus present in the organic layer. The lower aqueous layer was re-extracted under nitrogen with 1 part fresh MTBE. No cobalt appeared in the organic layer following the attempted extraction.

0.2g of 36% acetic acid in water were then added to the lower aqueous layer/MTBE mixture. A small amount of air was introduced to oxidise the remaining cobalt catalyst under a blanket of nitrogen. 23% of the remaining cobalt partitioned into the organic layer. Total cobalt recovery (initial water extraction plus partial oxidation and extraction) of cobalt was 83%. The organic layer was found to contain 0.9 wt% HPA, while the aqueous layer contained about 21.4 wt% HPA.

As can be seen by comparison of this result with Example 1, water extraction of HPA under CO significantly improved cobalt recycle in the organic layer. Moreover, some of the cobalt remaining in the aqueous phase could be recovered by partial oxidation and extraction with MTBE.

### Example 3

This example further illustrates the use of a carbon monoxide atmosphere to improve catalyst recovery for recycle after hydroformylation. 14g of EO were hydroformylated with 0.87g dicobaltoctacarbonyl, 0.14g of sodium acetate trihydrate, 1.5g toluene, 1.5g undecanol, 4.5g tetrahydrofuran and 146g MTBE, under 10.4 MPa (1500 psig) 3:1 H₂/CO at 80 °C. After 1.5 hr, the reaction mixture was found to contain 8.31 wt% HPA.

The reaction mixture was cooled to room temperature and extracted with 30g of deionised water under an atmosphere of 1.5 MPa (200 psig) CO. 72.2g of an upper organic layer containing 1638 ppmw cobalt were isolated. 33.4g of a lower aqueous layer containing 1040 ppmw cobalt. Cobalt recycle with the organic layer was 77%.

0.2g of 36% acetic acid in water were added to the lower layer, along with 30g of fresh MTBE solvent, 150 ml of ambient pressure air and 6.0 MPa (850 psig) CO. The mixture was stirred for 30 min. at 25 °C and then the phases were allowed to separate. 29.3g of upper organic layer containing 1158 ppmw cobalt were recovered. 32.lg of lower aqueous layer contained only 123 ppmw cobalt. Overall, 98% of the starting cobalt catalyst was recovered and recycled with MTBE solvent to the hydroformylation reaction.

### Example 4

A series of experiments was done to determine the effect of extraction under carbon monoxide, alone or as a CO/H₂ blend, on the recovery of cobalt following hydroformylation. Hydroformylations employed sodium acetate promoter in MTBE solvent under essentially the same reaction conditions as described previously.

Extraction conditions and results are shown in Table 1. As can be seen from the table, extraction under CO increased cobalt recycle in the organic layer following water extraction.

**Table 1**

| Extraction | | | | | | | |
|---|---|---|---|---|---|---|---|
| Gas | Extraction | | Temp. | wt OL | OL Co | AL Co | Co* |
| | MPa | (psig) | (°C) | wt AL | (ppm) | (ppm) | (%) |
| N₂ | 0.11 | (1) | 80 | 2.5 | 0 | 5900 | 0 |
| N₂ | 0.8 | (100) | 25 | 4.97 | 913 | 5400 | 46 |
| N₂ | 0.11 | (1) | 25 | 0.76 | 795 | 444 | 58 |
| N₂ | 0.8 | (100) | 25 | 2.22 | 901 | 1828 | 52 |
| 1:1 H₂/CO | ±0.12 | (±5) | 25 | 5.91 | 1664 | 1989 | 83 |
| CO | 0.8 | (100) | 25 | 2.24 | 433 | 368 | 73 |
| CO | 1.5 | (200) | 25 | 3.19 | 1893 | 1723 | 78 |
| CO | 1.5 | (200) | 25 | 1.85 | 2235 | 416 | 91 |
| CO | 2.2 | (300) | 25 | 2.16 | 1658 | 1040 | 77 |
| OL = organic layer AL = aqueous layer | | | | | | | |

| | | | | | | | |
|---|---|---|---|---|---|---|---|
| * Percent total cobalt retained in organic layer. | | | | | | | |

### Example 5

A series of experiments was done to determine the effect of extraction under carbon monoxide on the recovery of cobalt following hydroformylation.

Hydroformylations were conducted on 300 ml or 3.79 1 (1 gallon) scales in the presence of quaternary ammonium acetate ("ETHOQUAD" 2 C/11, a trademark for a bis(C₁₂₋₁₃ alkyl) (hydroxyethyl)methylammonium acetate) as lipophilic promoter (0.1 moles, relative to cobalt) at 80 °C in MTBE, and 2.3:1 to 3.0:1 H₂/CO (with total pressures given in Table 2). Reactions were restricted to formation of less than 10 wt% HPA prior to water extraction. Water extractions were carried out at 25 to 40 °C and 0.5 to 2.2 MPa (50 to 300 psig) CO, with varying amounts of water added to give organic/aqueous phase ratios of 1.5:1 to 4:1. As can be seen from Table 2, the use of a lipophilic ammonium salt promoter and extraction under CO enabled the recycle of 90% or more of the cobalt catalyst with the hydroformylation reaction solvent, while HPA was preferentially concentrated and extracted into the aqueous phase at a greater than 10:1 ratio. Thus, cobalt catalyst and HPA were efficiently separated. Moreover, Run 7 represented a recycle of catalyst from Run 6 (3.79 1 scale). For Run 7, a hydroformylation rate of 33 h⁻¹ was obtained, compared with a rate of 35 h⁻¹ in Run 6. This illustrates that the invention enables the majority of the catalyst to be recycled in essentially active form.

**Table 2**

| | Hydrof. | | % Co* | HPA wt% | HPA wt% |
|---|---|---|---|---|---|
| Run | MPa | (psig) | | AL | OL |
| 1 | 10.4 | (1500) | 91.4 | 12.1 | 1.1 |
| 2 | 10.4 | (1500) | 91.5 | 20.3 | 0.9 |
| 3 | 10.4 | (1500) | 90.4 | 21.7 | 1.2 |
| 4 | 10.4 | (1500) | 94.4 | 23.2 | 0.8 |
| 5# | 10.4 | (1500) | 81.5 | 17.7 | 1.4 |
| 6 | 19.1 | (2750) | 99.2 | 22.7 | 0.7 |
| 7# | 19.1 | (2750) | 92.1 | 24.8 | 1.6 |

| | | | | | |
|---|---|---|---|---|---|
| * Percent total cobalt retained in organic layer. | | | | | |
| # Recycled catalyst. | | | | | |

## Claims

1. A process for preparing 1,3-alkanediols and 3-hydroxyaldehydes by hydroformylating an oxirane with carbon monoxide and hydrogen in the presence of one or more Group VIII metal-based hydroformylation catalysts, and in the presence of an organic solvent, characterized in that the hydroformylation product is separated by extraction with an aqueous liquid under a carbon monoxide atmosphere.

2. A process as claimed in claim 1, wherein the oxirane is a hydrocarbyl-epoxide having of 2 up to 30 carbon atoms.

3. A process as claimed in claim 1, wherein the oxirane is ethylene oxide.

4. A process as claimed in any one of the preceding claims, wherein the solvent is an alcohol or ether according to formula (1)
R¹-O-R² (1)
in which R¹ is selected from hydrogen, a linear, branched, cyclic or aromatic C₁₋₂₀ hydrocarbyl or mono-or polyalkylene oxide, and R² is selected from a linear, branched, cyclic or aromatic C₁₋₂₀ hydrocarbyl, alkoxy or mono- or polyalkylene oxide, or R¹, R² and O together form a cyclic ether.

5. A process as claimed in the preceding claims, wherein the Group VIII metal is cobalt.

6. A process as claimed in any one of the preceding claims, wherein the reaction mixture comprises a promoter.

7. A process as claimed in any one of the preceding claims, wherein the level of 3-hydroxyaldehyde in the reaction mixture is maintained at less than 15 wt%.

8. A process as claimed in any one of the preceding claims, wherein the hydroformylation is carried out at a temperature within the range of 50 to 100 °C and a pressure within the range of 3.5 to 34.6 MPa.

9. A process as claimed in any one of the preceding claims, wherein the carbon monoxide atmosphere pressure is maintained within the range of 0.2 to 13.9 MPa.

10. A process as claimed in any one of the preceding claims, wherein the carbon monoxide atmosphere is a mixture of carbon monoxide and a gas selected from the group consisting of argon, hydrogen, nitrogen and methane.

11. A process as claimed in any one of the preceding claims, wherein the hydroformylation product is separated by addition of water in an amount to provide a water:mixture weight ratio within the range of 1:1 to 1:20.

12. A process as claimed in any one of the preceding claims, wherein the Group VIII metal-based catalyst is recycled.

13. A process as claimed in any one of the preceding claims, wherein the 3-hydroxyaldehyde is hydrogenated to yield the 1,3-alkanediol.

## Patentansprüche

1. Verfahren zur Herstellung von 1,3-Alkandiolen und 3-Hydroxyaldehyden durch Hydroformylieren eines Oxirans mit Kohlenmonoxid und Wasserstoff in Anwesenheit eines oder mehrerer Hydroformylierungskatalysatoren auf der Basis von Gruppe VIII-Metall und in Gegenwart eines organischen Lösungsmittels, dadurch gekennzeichnet, daß das Hydroformylierungsprodukt durch Extraktion mit einer wäßrigen Flüssigkeit unter einer Kohlenmonoxidatmosphäre abgetrennt wird.

2. Verfahren nach Anspruch 1, worin das Oxiran ein Hydrocarbylepoxid mit 2 bis 30 Kohlenstoffatomen ist.

3. Verfahren nach Anspruch 1, worin das Oxiran Ethylenoxid ist.

4. Verfahren nach einem der vorstehenden Ansprüche, worin das Lösungsmittel ein Alkohol oder Ether entsprechend der Formel (1)
R¹-O-R² (1)
ist, worin R¹ für Wasserstoff, lineares, verzweigtes, cyclisches oder aromatisches C₁₋₂₀Hydrocarbyl oder Mono- oder Polyalkylenoxid steht und R² für ein lineares, verzweigtes, cyclisches oder aromatisches C₁₋₂₀Hydrocarbyl, Alkoxy oder Mono- oder Polyalkylenoxid steht oder R¹, R² und O zusammen einen cyclischen Ether ausbilden.

5. Verfahren nach den vorstehenden Ansprüchen, worin das Gruppe VIII-Metall Kobalt ist.

6. Verfahren nach einem der vorstehenden Ansprüche, worin das Reaktionsgemisch einen Promotor umfaßt.

7. Verfahren nach einem der vorstehenden Ansprüche, worin der Gehalt an 3-Hydroxyaldehyd im Reaktionsgemisch auf unter 15 Gew.-% gehalten wird.

8. Verfahren nach einem der vorstehenden Ansprüche, worin die Hydroformylierung bei einer Temperatur im Bereich von 50 bis 100°C und bei einem Druck im Bereich von 3,5 bis 34,6 MPa ausgeführt wird.

9. Verfahren nach einem der vorstehenden Ansprüche, worin der Druck der Kohlenmonoxidatmosphäre im Bereich von 0,2 bis 13,9 MPa gehalten wird.

10. Verfahren nach einem der vorstehenden Ansprüche, worin die Kohlenmonoxidatmosphäre ein Gemisch aus Kohlenmonoxid mit einem aus der aus Argon, Wasserstoff, Stickstoff und Methan bestehenden Gruppe ausgewählten Gas ist.

11. Verfahren nach einem der vorstehenden Ansprüche, worin das Hydroformylierungsprodukt durch Zugabe von Wasser zur Ausbildung eines Gewichtsverhältnisses Wasser/Gemisch im Bereich von 1:1 bis 1:20 abgetrennt wird.

12. Verfahren nach einem der vorstehenden Ansprüche, worin der Katalysator auf Gruppe VIII-Metallbasis recycliert wird.

13. Verfahren nach einem der vorstehenden Ansprüche, worin der 3-Hydroxyaldehyd zur Ausbildung des 1,3-Alkandiols hydriert wird.

## Revendications

1. Procédé de préparation de 1,3-alcanediols et de 3-hydroxyaldéhydes par l'hydroformylation d'un oxiranne avec le monoxyde de carbone et l'hydrogène en présence d'un ou plusieurs catalyseurs d'hydroformylation à base de métaux du groupe VIII et en présence d'un solvant organique, caractérisé en ce que le produit de l'hydroformylation est séparé par extraction par un liquide aqueux sous une atmosphère de monoxyde de carbone.

2. Procédé suivant la revendication 1, caractérisé en ce que l'oxiranne est un hydrocarbyl-époxyde possédant de 2 jusqu'à 30 atomes de carbone.

3. Procédé suivant la revendication 1, caractérisé en ce que l'oxiranne est l'oxyde d'éthylène.

4. Procédé suivant l'une quelconque des revendications précédentes, caractérisé en ce que le solvant est un alcool ou un éther répondant à la formule (1) :
R¹-O-R² (1)
dans laquelle R¹ est choisi parmi l'hydrogène, un radical oxyde de mono- ou polyalkylène ou un radical hydrocarbure en C₁ à C₂₀, linéaire, ramifié, cyclique ou aromatique et R² est choisi parmi un radical oxyde de mono- ou polyalkylène, alcoxy ou hydrocarbyle en C₁ à C₂₀, linéaire, ramifié, cyclique ou aromatique, ou bien R¹, R² et O forment ensemble un éther cyclique.

5. Procédé suivant les revendications précédentes, caractérisé en ce que le métal du groupe VIII est le cobalt.

6. Procédé suivant l'une quelconque des revendications précédentes, caractérisé en ce que le mélange réactionnel comprend un promoteur.

7. Procédé suivant l'une quelconque des revendications précédentes, caractérisé en ce que le taux de 3-hydroxyaldéhyde dans le mélange réactionnel est maintenu à moins de 15% en poids.

8. Procédé suivant l'une quelconque des revendications précédentes, caractérisé en ce que l'on entreprend l'hydroformylation à une température comprise entre 50 et 100°C et sous une pression comprise entre 3,5 et 34,6 MPa.

9. Procédé suivant l'une quelconque des revendications précédentes, caractérisé en ce que la pression atmosphérique du monoxyde de carbone est maintenue dans la plage de 0,2 à 13,9 MPa.

10. Procédé suivant l'une quelconque des revendications précédentes, caractérisé en ce que l'atmosphère de monoxyde de carbone est constituée d'un mélange de monoxyde de carbone et d'un gaz choisi dans le groupe formé par l'argon, l'hydrogène, l'azote et le méthane.

11. Procédé suivant l'une quelconque des revendications précédentes, caractérisé en ce que le produit de l'hydroformylation est séparé par l'addition d'eau en une proportion telle que l'on obtienne un rapport pondéral eau:mélange qui varie de 1:1 à 1:20.

12. Procédé suivant l'une quelconque des revendications précédentes, caractérisé en ce que le catalyseur à base de métal du groupe VIII est recyclé.

13. Procédé suivant l'une quelconque des revendications précédentes, caractérisé en ce que l'on hydrogène le 3-hydroxyaldéhyde pour obtenir le 1,3-alcanediol.
